# EUROPEAN PATENT APPLICATION

(11) **EP 1 787 637 A2**
(43) Date of publication of application: **23.05.2007**
(21) Application number: 06380231.8
(22) Date of filing: 18.08.2006
(51) Int. Cl.: A61K 9/06, A61K 33/30, A61K 36/54, A61K 31/4166, A61K 36/48, A61K 36/23, A61K 36/886, A61P 17/02

(54) **Composition for veterinary use**

(30) Priority: 18.08.2005 ES 200502114
(71) Applicant: Caballero Gobern, Francisco, 25711 Montferrer (Lleida) (ES); Foradada, Mercè, 25711 Montferrer (Lleida) (ES)
(72) Inventor: Caballero Gobern, Francisco, 25711 Montferrer (Lleida) (ES); Foradada, Mercè, 25711 Montferrer (Lleida) (ES)

(57) **Abstract**

Composition for veterinary use as semisolid preparation to be used topically comprising at least one astringent, one rubefacient, one antipruritic agent, one cicatrizing agent, one antiseptic and one emollient suitable for veterinary use where the astringent is zinc oxide or a suitable salt of zinc; the rubefacient and antipruritic agent is a natural, a semi-synthetic or a synthetic derivative of Cinnamomum camphora; the cicatrizing agent is 5-ureidohydantoin [2,5-dioxo-4-imidazolidynil] urea; the antiseptic is benzalconium chloride or a suitable antiseptic into fatty emollient base providing the appropriate consistent for the use and containing at least one oil from vegetal origin and/or an oil from animal origin.

The composition can be used in the prophylaxis, cicatrisation and treatment of wounds, lacerations, chaps and ulcerations on the skin of the animals forming a lubricating film protecting and impermeable on the affected area.

## Description

The invention relates to compositions suitable for veterinary use in pets and livestock to be used to the prevention and treatment by topical way of wounds, lacerations, ulcerations and chaps of the skin, preferably those located in the udders of cows, sheep and goats.

The treatment of wounds on the skin of animals rise some difficulties derived from their life habits and areas where are living, specially those that live together in farms or under some circumstances that avoid an exhaustive monitoring by their take carer, making difficult a suitable attention of the skin injuries when these are produced, their disinfection, cicatrisation and the protection of the affected parts against external agents as micro organisms or parasites that can damage the curative process.

In the veterinary field is interesting to have available specific products for the prevention and treatment of wounds on the skin of animals, designed with the aim of having an appropriate adherence on the affected area, that the formulation characteristics avoid an easy elimination of the product by the animal itself or by friction or contact of affected area, that the formulation provide an appropriate protection and impermeable properties against external contamination agents and the moisture, with the goal of providing a quick cicatrisation and reparation of the affected tissues, an appropriate antisepsis, reducing therefore the possibilities of complication, the perpetuation of the wound and improving their prognostic. This interest is remarked when the affected area is located in the udders of cows, sheeps and goats.

The tissue of the udder of cows, sheep and goats is a very sensible tissue that can undergo lesions, some of them and frequently, derived from the milking operations or lactation of animals. These lesions are usually difficult of healing, due to the tissue characteristics, moisture conditions and hygiene of the farms, as well as the location of the affected area.

Some existing products are intending to work out this problem, but their efficacy is limited because are not including in their composition some ingredients that can be important for the prevention and cicatrisation of this kind of tissue for the appropriate consistence of the formulation as well as for an adequate protection of the tissue to be treated in the farm conditions after the product application.

The main existing products for udder treatment, their goal are the seal and disinfection of the tead after milking operations, often incorporating a disinfectant and a viscosity agent to allow the adherence of the product to the tead. In the veterinary praxis, for the treatment of the lesions of the tead skin are used aerosol products based on tetracyclines, that although are effective when are used for skin lesions not located in the udder, their application on this area is not always solving the problem and additionally the use of an antibiotic is limiting when the animals are food producers. The differences related to the product of this invention are essentially that the application of the mentioned products for seal and disinfection is carried out by immersion of the teat in a teat bath, not being indicated their use in other areas of the udder, other essentially difference is that those products has a higher content of water over fifty percent in the main cases and are for this reason easily removable by water contact or moisture of the farms, additionally avoiding the suitable drying of the wound. The water presence can help the micro organisms proliferation as well as the formation of chaps on the skin, specially in the cold season of the year. An other essentially difference is that the formed film is not impermeable to water, external contaminants or sun radiation. An other difference is that are little effective in the treatment of ulcerative lesions of the skin because of their limited cicatrising power. An other essentially difference is that their consistence is liquid and their formulation is not based on zinc compound at high concentrations.

The composition of this invention is not applied by means of teat bath and it is not exclusive for this area of the udder, their application is by means of the extension upon any affected area of the udder or any other part of the animal skin, acting like a occlusive apposite , resistant an impermeable to the water, exerting the effect of a physical barrier, resulting a composition prepared in semisolid form for topical use, disinfecting, highly cicatrising, antipruritic and protector of the affected area.

Bearing in mind the alimentary safety, the ingredients contained in medicines to be used in food producing animals must be accepted in an explicit way for this use, not being acceptable any pharmaceutical composition suitable for other uses as human or pet treatment. The ingredients included is this invention belong to the group of ingredients authorised for medicines to be use in livestock animals.

### Explication

The formulation of this invention needs an specific composition and suitable incorporation of ingredients for obtaining a consistence to allow a right protection and tissue adherence to the animal to be treated, being this one of the characteristics for the well working of the product and their application, under the basis to allow a film formation protecting and lubricating that remains in the tissue to be treated, overcoat for the application on the udders of cows, sheep and goats.

The formulation is presented under the pharmaceutical dosage form of semisolid preparation for topical use, preferably in the form of ointment or paste.

One of the innovations of the invention, is the fact to have available a formulation for use in animals that allow the formation of an impermeable film persistent on the skin of the animal, whose application has not be repeated with excessive frequency due to absorption of the product or their spontaneous elimination when is in touch with wet places as could be the floor of farms or pastures. It is avoided on this way that the affected area remains uncovered and be susceptible to be contamined by micro organisms, minimize the repeated intervention of the carer, with the subsequent reduction of production costs. It is specially useful and effective when is applied on skin lesions located on the udder where it is showed high reparation action of the affected tissue.

The composition of this invention is also especially useful for the treatment of infosura or laminitis, illnesses of the animal hoof, that could require the chirurgic treatment and due to their location in constant contact with the floor is necessary a physical barrier impermeable disinfecting, highly cicatrising and protecting of the affected area after the chirurgic treatment.

The use of medicines in food producing animals not only requires the presence of authorised ingredients in the formulation in addition usually is necessary to establish a withdrawal period of the medicine before the food production, having into account the risk of the medicines and their pharmacokinetic that could represent economical loses due to the rejection during the treatment and until the end of the withdrawal period the produced products or remove the treated areas in the even of sacrifice. The ingredients used in this composition are authorised for food produced animals and due to their characteristics is not necessary to establish any withdrawal period. This is an advantage because the treatment can be done at the same time of the food extraction, being this an advantage in the case of milk productions where animals are milked twice a day.

The goal is to have available a full composition that contributes in a effective way to the protection and healing of the affected skin that may require only one application if the affection is slight or is used as prophylactic or repeated applications in the even of severe wounds. As general rule a low frequency of application of the composition on the wounds can be sufficient for their cicatrisation and healing being this other of the characteristics of the formulation.

The curative process is facilitated by the action of the antiseptic that maintains the lesion free of micro organism by the action of the cicatrising agent that helps the re epitelisation of the damaged area and by the rubefacient agent that stimulates the local blood irrigation of the affected area, contributing to the process of defence of the affected tissue and their regeneration. The antipruritic and calming action is other of the characteristics of the composition because of their contribution to the alleviate and welfare of the animal during the curative process, but essentially because acts avoiding the scratching of animal and the erosion that exerts on the damaged skin.

The healing of the lesions in dogs and cats that appears as consequence of allergic dermatitis caused by ecto parasites as fleas and chronified by the scratching of the animal on the affected area is produced efficiently by the application of the composition of this invention. The presence of an antipruritic agent avoid first the itching sensation reducing therefore the scratching and on the other hand, the consistence of the composition create a film that the animal can not remove by means of scratching or licking, that is not accessible to the ecto parasites and this infect and cicatrise the lacerated tissue, being this other of the main application of the composition. For this application one improved form of the composition of this invention is that is comprising between a 1 and a 5 % of boric acid or their salts as antiseptic, preferred for this application because of the insecticidal effects of boric acid and borates in addition of the antiseptic effect being an advantage in the contribution to the elimination of the etiological agent.

To have a suitable barrier effect the composition is comprising a high concentration of 5-oxide or a suitable salt of zinc, preferably higher than 15 % and more preferably higher than 23 % having the composition a consistent excessively solid that could avoid the proper application. The presence of alcamphor or a cinnamomum camphora derivative in addition of the rubefacient and antipruriginos effect is providing a decrease of the consistence making it suitable to allow their extensibility and application being this other of the relevant characteristics of this invention.

The emollient basis contributes to the protection, lubrication and impermeabilisation of the affected area, allowing to adjust the consistency of the composition in order to be appropriate for their application as well as for remaining an effect in the side of action.

The composition of this invention is related to a semisolid form to be administered by topical way suitable for veterinary use comprising the following ingredients: preferably zinc oxide or a suitable salt of zinc, like zinc stearate, zinc acetate, zinc chloride, zinc gluconate, zinc oleate or a mixture of them; preferably benzalconium chloride or a suitable antiseptic like phenol, chlorocresol, benzetonium chloride, cetrimide, hexetidine, chlorhexidine acetate, chlorhexidine gluconate, iodated povidone, sorbic acid, potassium sorbate, boric acid, sodium borate, lactic acid or a mixture, preferably 5-ureydohydantoine [2,5-dioxo-4-imidazodinil] urea or a derivative; preferably alcamphor or a derivative of Cinnamomum camphora obtained by natural, semi synthetic or synthetic sources; an emollient base comprising one or more of the following ingredients: a mineral oil, a vegetal oil, olive oil, almond oil, peanut oil, soybean oil, castor oil, coconut oil, calendula oil, retinoic acid, vitamin A palmitate, isopropyl miristate, isopropyl palmitate, waxes, medium chain tryglicerides, karite oil or a mixtures of them and/or a oil or fat of animal origin, preferably fish oil and/or lanoline.

The emollient base comprise a substance or mixture of thickening substances or modifiers of viscosity selected of the group formed by aluminium stearate, starch, carboxymethylcellulose, methylcellulose, ethylcellulose, hydroxypropylcellulose, , poliethyleneglicols, polivinylpirrolidone, and xantan gum.

The emollient basemay incorporate propileneglicol and/or glycerine.

According the described qualitative composition, other of the characteristics of the formulation is the following quantitative composition expressed in percentage in weight, and is coprising: Zinc oxyde or a salt of zinc or a mixture of them between 15 and 30 percent, preferably 25 percent; one antiseptic, preferably benzalconium chloride between 0,01 and 1 percent, preferably 0,05 percent; ureidohydantoin [2,5-dioxo-4-imidazolidinil]urea between 0,2 and 5 percent preferably 2 percent; alcamphor or a Cinnamomum camphora derivative between 1 and 10 percent and an emollient base that is acting as vehicle of the previous ingredients and together with the previous ingredients provide the appropriate consistency for a suitable use.

According to the expressed composition, the emollient base comprises a base of mineral oil, preferably liquid paraffin, gelified with aluminium stearate in a quantity comprised between 1 and 5 percent, preferably a 3 percent, or a mixture of one or more of the ingredients selected from the group comprising: (vaselin filante, olietilenglicol, liquid paraffin, a vegetal oil like peanut oil, olive oil, almond oil, castor oil, bee wax, carnauba wax, medium chain triglicerides, isopropyl palmitate, isopropyl miristate, an animal oil, starch, olietilenglicol, polivinylpiroolidone and lanolin.

The composition of this invention can be filled in any kind of container, and is not limited to any kind of specific container described thereafter with detail, which must be considered only as an example.

Other of the characteristics of the invention is the low content in water that is comprised between 0 0and 10 percent, preferably lower than 1 percent and more preferably lower than 0,05 percent.

According other of the characteristics, the composition of this invention may incorporate stabilising agents, modifiers of organoleptic characteristics, or preservative or a mixture of them.

Between the modifiers of organoleptic characters, are preferred the modifiers of colour and flavour.

According other of the characteristics of the compositon, between the preservatives are preferred those with activity against fungi, such as methyl p-hidroxybenzoate, propyl p-hidroxybenzoate, ethyl p-hidroxybenzoate or their mixtures.

According other of the characteristics, the formulation contains an antioxidant agent, preferably buthylhidryanisol (BHA), buthylhidroxytoluene (BHT), tocopherol or a mixture of them.

According other of the characteristics of the invention an improved form of the same is comprising the addition of vegetal extracts preferably, balsam of Peru, centella asiatica or an extract, aloe or an extract or a mixture of all them.

According other of the characteristics of the invention, is comprising one or more thickening agents selected from the group of aluminium state, pliethyleneglicol, waxes, starch, wheat starch, corn starch, rice starch, potato starch, carboxymethylcellulose, hidrxypropylcellulose, ethylcellulose, methylcellulose, xantan gum in a concentration comprised between 1 and 35 percent in weight.

The way of dosification will be variable in function of the affected area. As general rule the formulation will be applied covering totally all the affected area forming a thick film.

According other of the characteristics of the invention, has an effect of sun filter to protect the affected area of the sun radiation.

### Method of preparation

The composition of this invention is prepared by mixture of the ingredients, heating in order to improve the mixture and filling processes.

Fro the preparation of a pilot batch it was proceed according the following master method:
a) In a tank suitable for the preparation of semisolid dosage forms, provided with heating, quick stirring, slow stirring, triturator, perfectly clean and sterilised, is incorporated a mixture of the oils of the emollient base.
b) The mixture is heated until liquid state.
c) Thickening agents are added
d) The solids previously sieved, are added sequentially, homogenizing with quick stirring.
e) The triturator is switched on until a fine and homogeneous mixture.
f) The temperature is lowered between 40- 50 °C and is added the antiseptic, allowing and appropriate homogenisation.
g) The mixture is maintained under low stirring and temperature around -40°C.

The product is filled in monodose or multidose containers, in aluminium tubes, polipropylene tubes or polipropylene flasks.

The described method is just one of the possibles for the preparation of the composition, being valid any method consisting in the sequential addition of the ingredients individually or premixed, and further agitation, using mechanic energy or heat energy, or both concomitantly, being this last the preferred way, because of their better operativity.

Although pharmacopoeias, are not requiring sterility for compositions for topical use, because the substrate where are used is not free of microorganisms, the application of formulations or apposites upon open wounds is recommended to be carried out in the better conditions of asepsis in order to not add more contamination, for this reason can be used methods of aseptic production for the attainment of the product, methods of microbiological reduction or final sterilisation. Between the preferred methods to reduce or eliminate the bioburden of the composition of this invention, are those using starting materials previously sterilised and are prepared in installations and equipes free of contamination or those methods using heat, irradiation and or gasification in any part of the process or at the end of the same. Moreover, the presentation in monodose containers may contribute to a better handling of the product and warranty of asepsis when different applications are necessary.

### Examples of composition:

In the following table are exposed different formulations of the composition for veterinary use as example:

**Table 1**

| *Examples of the compositions for veterinary use (expressed as percentage in weight)* | | | | | | |
|---|---|---|---|---|---|---|
| **Ingredient** | **A** | **B** | **C** | **D** | **E** | **F** |
| Benzetonium chloride | 0,00 | 0,00 | 0,00 | 0,02 | 0,00 | 0,00 |
| Phenol | 1,00 | 0,01 | 0.00 | 0,00 | 0.00 | 0,00 |
| Boric acid | 0,01 | 0,00 | 0,01 | 0,00 | 0,01 | 3,00 |
| Lactic acid | 0,01 | 0.05 | 0,00 | 0,01 | 0,00 | 0,00 |
| Benzalconium chloride | 0,01 | 0,10 | 0,05 | 0,10 | 0,00 | 0,00 |
| Cetrimide | 0,00 | 0,00 | 0,00 | 0,00 | 0,50 | 0,00 |
| Sorbic acid | 0,00 | 0.00 | 0,00 | 0.00 | 0,01 | 0,01 |
| Triclosan | 0.01 | 0,00 | 0,00 | 0,00 | 0.01 | 0,00 |
| Benzil alcool | 0,01 | 1,00 | 0,00 | 1,00 | 0,50 | 0,00 |
| Hexetidine | 0,01 | 0,01 | 0,00 | 0.00 | 0,00 | 0,00 |
| Chlorhexidine acetate | 0,00 | 0,01 | 0,00 | 0,00 | 0,00 | 0,00 |
| Chlorhexidine gluconate | 0,00 | 0,01 | 0,00 | 0,00 | 0,01 | 0,00 |
| Povidone iodate | 0,00 | 0,00 | 0,00 | 0,00 | 0,01 | 0,00 |
| Chlorocresol | 0,01 | 0,01 | 0,00 | 0,00 | 0,01 | 0,50 |
| Glycerine | 1,00 | 0,50 | 0,01 | 0,50 | 0,50 | 0,00 |
| Propyleneglycol | 1,00 | 0,50 | 0,10 | 0,50 | 0,50 | 0,00 |
| Polyethyleneglycol | 4,00 | 0,50 | 0,20 | 0,10 | 1,00 | 3,00 |
| 5-ureidohydantoin[2,- | 1,50 | 1,50 | 2,00 | 2,00 | 1,50 | 2,00 |
| dioxo-4-imidazolidinyl]urea | | | | | | |
| Bálsam of Perú | 0,00 | 0,01 | 0,00 | 1,00 | 0,00 | 0,01 |
| Extr. *Centella Asiática* | 0,00 | 0,01 | 0,00 | 0,50 | 0,00 | 0,00 |
| Etr. *Aloe vera* | 0,00 | 0,01 | 0,00 | 0,50 | 0,00 | 0,00 |
| Karité oil | 0,50 | 0.50 | 0,01 | 0,50 | 0,00 | 0,01 |
| Peanut oil | 0,00 | 1,00 | 0,01 | 1,00 | 0,00 | 0,01 |
| Triglicerides | 2,00 | 1,00 | 0,50 | 2,00 | 0,00 | 1,00 |
| Calendula oil | 2,00 | 0,01 | 0,02 | 2,00 | 0,00 | 0,50 |
| Olive oil | 0,00 | 1,00 | 0,05 | 0,00 | 3,00 | 0,00 |
| Castor oil | 1,00 | 1,00 | 0,01 | 0,00 | 0,05 | 0,00 |
| Almond oil | 2,00 | 1,00 | 4,00 | 3,00 | 1,50 | 4.00 |
| Fish oil | 2,00 | 0,00 | 1,00 | 0.01 | 0,00 | 0.00 |
| Vit A palmitate | 0,05 | 0,05 | 0,01 | 0,01 | 0,00 | 0,05 |
| BHT | 0,05 | 0,05 | 0,00 | 0,10 | 0,01 | 0,05 |
| BHA | 0,05 | 0,05 | 0,00 | 0,10 | 0,01 | 0,05 |
| Tocopherol | 0,5 | 0,50 | 1,00 | 0,50 | 0,01 | 0,50 |
| Zinc stearate | 15,00 | 10,00 | 0,00 | 5,00 | 3.00 | 0,01 |
| Zinc gluconate | 2,00 | 0.00 | 0,00 | 1,00 | 0.01 | 0,00 |
| Zinc oleate | 2,00 | 3.00 | 0,00 | 1,00 | 0,01 | 0,01 |
| Zinc oxide | 0,00 | 15,00 | 25,00 | 10,00 | 22,79 | 22,00 |
| Zinc sulphate | 0,01 | 0.00 | 0,00 | 0,00 | 0,00 | 0,00 |
| Zinc chloride | 0,00 | 0,00 | 0,00 | 0,00 | 0,00 | 0,01 |
| Lanoline | 1,00 | 4,00 | 0,01 | 2,00 | 2,00 | 1,00 |
| LiquidParafine | 26,42 | 2,00 | 0,01 | 2,00 | 25,51 | 1,00 |
| Vaseline | 25,42 | 23,30 | 34,91 | 32,05 | 27,50 | 31.72 |
| Aluminio stearate | 3,00 | 3,00 | 1,00 | 1,70 | 3,00 | 1,50 |
| Rice starch | 0,00 | 15,00 | 15,00 | 25,00 | 0,00 | 11,00 |
| Potato starch | 0,00 | 5,00 | 0,01 | 0,00 | 0,00 | 11,00 |
| Corn starch | 0,00 | 5,00 | 0,01 | 0,00 | 0,00 | 0,50 |
| Wheat starch | 0,00 | 0,10 | 10,00 | 0,00 | 0,00 | 0,50 |
| Water | 0,30 | 1,00 | 0,01 | 0,01 | 0,01 | 0,00 |
| Methyl celulose | 2,00 | 0,01 | 0,01 | 0,50 | 0,01 | 0,01 |
| Hidroxipropylcelulose | 0,05 | 0,01 | 0,00 | 0,00 | 0,01 | 0,00 |
| etylcelulosa | 0,05 | 0,01 | 0,00 | 0,00 | 0,01 | 0,01 |
| Camphora | 4,00 | 3,00 | 5,00 | 4,20 | 7,00 | 5,00 |
| Colorant agent | 0,00 | 0,05 | 0,01 | 0,05 | 0,00 | 0,01 |
| Parfum | 0,00 | 0,10 | 0,01 | 0,01 | 0,00 | 0,01 |
| Methylparaben | 0,02 | 0,02 | 0,02 | 0,02 | 0,00 | 0,01 |
| Propylparaben | 0,01 | 0,01 | 0,01 | 0,01 | 0,00 | 0,01 |
| **TOTAL** | **100** | **100** | **100** | **100** | **100** | **100** |

### Evaluation of the efficacy

An experimental assay including animals form different farms of milking cows from the Pirineo Leridano, with the aim of determine the behaviour of the formulation in animals.

The main goal of the study is to show that the application of the composition of this invention significantly reduces the lesions that are produced in the skin of the animals, and more specifically those occurred in the studied species. Other goals of the study consisted in to show that the product has a safe behaviour for the treated animals, demonstrating an easy, and operative application of the formulation.

From 4 different farms, the responsible Vet selected with experimental purposes a number of 15 milking cows with ulcerations on the udder of diverse intensity and that could be susceptible to be treated with the composition of this invention. Eight of the selected animals had been previously by the carer or Vet to other treatments without success and without observing a significant improvement.

A criterion of evaluation of the lesions was established, based in an index from O to 4 for the following parameters evaluated: erithema, suppuration, oedema, size and deepness. Being the value 0 inexisting or not appreciable, 1 slight, 2 moderated, 3 severe, 4 very severe.

Application of the product: for this assay it was used a composition according to the pattern of the formulation in the example C and the described method of preparation. The composition was applied daily on the wound previously cleaned, directly from the container (tube) extending over all the surface of the wound with an spatula previously sterilised, on this way the wound was covered by a thick layer of the formulation forming a film that remained adhered until the next application. The treated animals was identified and reared in the same previous conditions with the other animals of their respective farms. The milk produced by the animals included in the study was rejected during the study period.

It was assigned the numeration from 1 to 8 for the animals that had previously been treated. The animals from 9 to 15 had not received any treatment.

The animals were examined at the start of the study, before the application of the treatment with the composition of this invention. The results are showed in the table 2. After 10 days of the application of the product the lesions were evaluated again, obtaining the results showed in the table 3.

None of the animals participating in the study had been excluded for death or intolerance to the treatment reasons.

**Table 2: Evaluation of the wounds before the treatment**

| | **Size** | **Erythema** | **Oedema** | **Depth** | **Supuration** |
|---|---|---|---|---|---|
| **Cow** 1 | 2 | 1 | 1 | 2 | 1 |
| **Cow 2** | 3 | 1 | 1 | 1 | 0 |
| **Cow 3** | 2 | 2 | 2 | 2 | 1 |
| **Cow 4** | 4 | 2 | 2 | 2 | 2 |
| **Cow 5** | 2 | 0 | 0 | 3 | 0 |
| **Cow 6** | 2 | 0 | 0 | 1 | 0 |
| **Cow** 7 | 4 | 2 | 2 | 1 | 2 |
| **Cow 8** | 3 | 1 | 0 | 1 | 0 |
| **Cow 9** | 2 | 1 | 1 | 2 | 1 |
| **Cow 10** | 2 | 0 | 0 | 1 | 0 |
| **Cow 11** | 3 | 1 | 1 | 2 | 1 |
| **Cow 12** | 2 | 0 | 0 | 3 | 0 |
| **Cow 13** | 2 | 0 | 0 | 1 | 0 |
| **Cow 14** | 2 | 2 | 2 | 2 | 2 |
| **Cow 15** | 2 | 0 | 0 | 1 | 0 |

a) For the determinations of the extension of the wound was measured the main axis of the same and from the product of the obtained measures it was attained de value of the surface. It was considered ulcerations of slide grade (1) those lesions whose surface was less than 20 cm², moderated grade lesions (2) those situated between 20 and 40 cm², severe grade (3) those situated between 40 to 80 cm², very severe grade those lesions whose surface was higher than 80 cm² .
b) In all the cases it was observed an statistical improvement (P<0.05) of the wounds compared with their situation before the treatment and also in comparison with the other methods of treatment previously used in the animals 1 to 8. It was not observed infection after the application of the treatment indicating an appropriate control of micro organisms. The improvement was notable and the total healing after the application of the product in most of the wounds. Those wounds whose healing was not total required further application of the same treatment until the total recuperation of the wound

The product was well tolerated and in any case it was observed an abnormal behaviour of the animals or symptoms of local or systemic toxicity.

**Table 3 Evaluation of the wounds after the treatment**

| ***** | **Size** | **Erythema** | **Oedema** | **Depth** | **Supuration** | **Recuperation** |
|---|---|---|---|---|---|---|
| **Cow** 1 | 0 | 0 | 0 | 0 | 0 | Total |
| **Cow 2** | 0 | 0 | 0 | 0 | 0 | Total |
| **Cow 3** | 0 | 0 | 0 | 0 | 0 | Total |
| **Cow 4** | 2 | 1 | 1 | 1 | 0 | Significant |
| **Cow 5** | 0 | 0 | 0 | 0 | 0 | Total |
| **Cow 6** | 0 | 0 | 0 | 0 | 0 | Total |
| **Cow 7** | 2 | 1 | 1 | 1 | 0 | Significant |
| **Cow 8** | 0 | 0 | 0 | 0 | 0 | Total |
| **Cow 9** | 2 | 0 | 0 | 1 | 0 | Significant |
| **Cow 10** | 1 | 0 | 0 | 0 | 0 | Total |
| **Cow 11** | 0 | 1 | 1 | 1 | 0 | Significant |
| **Cow 12** | 0 | 0 | 0 | 0 | 0 | Total |
| **Cow 13** | 0 | 0 | 0 | 0 | 0 | Total |
| **Cow 14** | 0 | 0 | 0 | 0 | 0 | Total |
| **Cow 15** | 0 | 0 | 0 | 0 | 0 | Total |

It was not observed nor oedema neither erythema except in 3 of the treated animals that presented a slide redness and swelling of the perimeter of the wound.

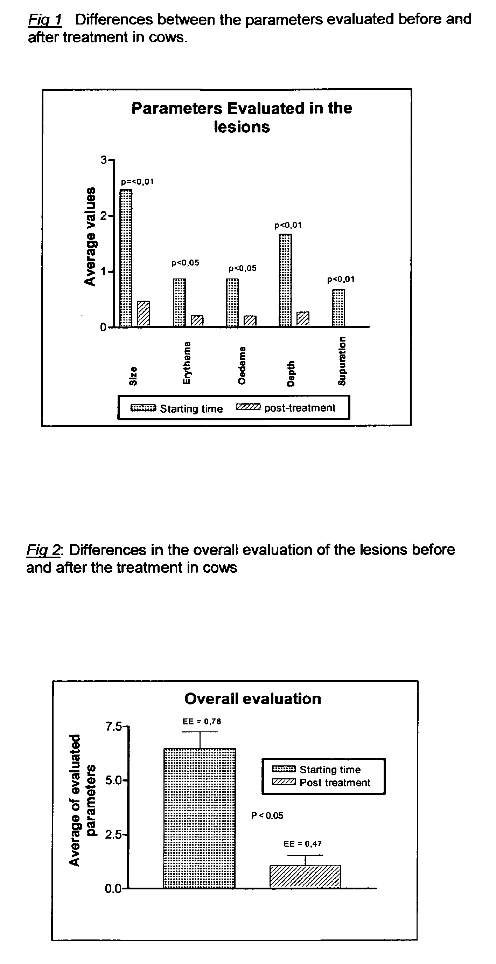

In the figure 1 and 2 are showed as graphic a summary of the results obtained on this study.

Study in dogs and cats: With experimental aim it was studied the action of the composition following the pattern of the formulation of the example C of this invention in an assay on 5 dogs of different races. The selected dogs presented allergic dermatitis located in different parts of the body, probably caused by ecto parasites. The dogs 1 and 5 presented the affection in the ventral abdomen, the dog 2 in the base of the ear, the dogs 3 and 4 in the area of the tail. The dogs showed nervous with frequent scratching on the affected area causing wounds on the same that in some case was treated by the carer with conventional antiseptics as iodated povidone. The affected areas were alopecic probably for the constant scratching of the animal.

It was applied a thick layer on the affected area that in the initial moments, after the application, the animals tried to remove by means of scratching or licking without success due to the consistence and impermeability of the composition. After the first moment, it was observed relief in the animal disappearing the initial nervous state. The frequency of licking and scratching decreased significantly and in an average time of three days after the application the lesions were cicatrised disappearing the erythema allowing the regeneration of the hair of the treated area.

In all the cases just one application was enough.

The same composition was applied with the experimental purposes in a litter of 4 cats with veterinary diagnostic of allergic dermatitis probably caused by ecto parasites. The results were the total remission of the observed symptoms and absence of any toxicity on the animals.

These results demonstrated the utility of the composition of this invention for the treatment of dermatitis in pets, caused by ecto parasites. This activity could be increased adding boric acid as antiseptic agent due their antiparasitic properties, following the pattern of formulation described in example F.

## Claims

1. A composition for veterinary use comprising:
a) zinc oxide, a salt of zinc or a mixture in an amount between the 15 and 30% of the total weight of the composition.
b) An antiseptic in an amount between 0.01 and 5% of the total weight of the composition.
c) A cicatrising agent in an amount between 0.01 and 5% of the total weight of the composition.
d) A rubefacient and antipruriginous in an amount of 0.01 and 10% of the total weight of the composition.
e) A thickening agent in an amount between 1 and 30% of the total weight of the composition.
f) An emollient base in an amount between 30 and 80% of the total weight of the composition.
g) Water in an amount lower than 10% of the total weight of the composition.

2. The composition according the claim 1 **characterised** wherein the rubefacient, antiprurigenous is camphora or a Cinnamomum camphora obtained by natural, semi synthesis or chemical synthesis, means or a mixture of them.

3. The composition of the claim 1 wherein the antiseptic is benzalconium chloride in an amount comprised between 0.02 and 5%.

4. The composition of the claim 1 wherein the thickening agent comprises one or more of the compounds of the group formed by Aluminium stearate, corn starch, potato starch, rice starch, wheat starch, carboximethilcelulose, ethilcelulose and polyethyleneglycol.

5. The composition of the claim 1 wherein the water content is lower than 1% of the toal weight.

6. A composition according any of the claims 1 to 5 that is applied on the wounds of animals belonging to the group of bovine, equine, swine, ovine, birds, cows, calf, pigs, horses, mules, donkeys, zebras, sheeps, goats, rodents, cats, dogs, ornamental birds, exotic birds, reptiles, poultry, ducks, rabbits, canary, parrots, parakeets, cockatoo.

7. A composition according any of the claims 1 to 5 wherein is applied on the udders of cows, sheeps and goats.

8. A composition according any of the previous claims wherein the withdrawal period in livestock animals is 0 days.

9. A method of prophylaxis and treatment of wounds of the skin of animals that comprises the step of the application of an effective amount of the composition according any of the claims 1 to 5 on the area to be treated.

10. The method of the claim 9 wherein the area to be treated is the udder or the hoof of the animal

11. The method of the claim 9 wherein the composition is extended on the affected area forming a protecting film.

12. The use of a composition according any of the claims 1 to 5 for the preparation of products to be used in the protection, cicatrisation, disinfection or treatment of wounds of the skin of the animals.

13. The use of a composition according any of the claims 1 to 5 wherein is added a colouring agent, perfume, a preservative or a mixture of them.

14. A composition according any of the claims 1 to 5 wherein is added an antioxidant.

15. The composition according the claim 14 wherein the antioxidant is buthylhidroxitoluene (BHT), butfylhidroxianisol (BHA), tocopherol or a mixture of them.

16. A method of manufacture of the composition according any of the claims 1 to 5 that comprises the mixture by means of stirring of the mentioned ingredients and the use of heat.

17. The method of the claim 16 wherein the filling is carried out by use of heat.

18. The method of the claim 16 wherein the product is prepared aseptically.

19. The method of the claim 16 wherein the product undergoes a process of final sterilisation or reduction of bioburden by means of use of heat, irradiation or gases.

20. A composition according any of the claims 1 to 5 that comprises the addition of balsam of Peru, Centella asiatica or an extract, aloe or an extract, or a mixture of all them.

21. A composition according any of the claims 1 to 5 **characterised** to be used for the treatment or prophylaxis of inespecific dermatitis or caused by ecto parasites in dogs and cats.

22. A composition for veterinary use for topical administration comprising the following mixture
a) Zinc oxide or a zinc salt or a mixture of both in an amount between 15 and 30% of the total weight of the composition wherein the salt of zinc is one or more of the compounds selected from the group consisting in stearate of zinc, oleate of zinc, zinc chloride, zinc sulphate, zinc gluconate and zinc acetate.
b)An antiseptic in an amount between 0.01 and 5% of the total weight of the composition wherein the antiseptic is one or more of the compounds selected from the group consisting in: benzetonium chloride, benzalconium chloride, cetrimide, benzilalcohol, chlorocresol, hexetidine, triclosan, chlorhexidine, acetate, chlorhexidine gluconate, iodate povidone, sorbic acid, sorbate, lactic acid, phenol, boric acid, sodium borate or a mixture of them.
c)A cicatrising agent in an amount between 0.1 and 5% of the total weight of the composition wherein the cicatrising is 5-ureidohydantoin [2,5-dioxo-4-imidazolidinil] urea.
d)A rubefacient, an antipruriginous in an amount between 0.001 and 10% of the total weight of the composition.
e) A thickening agent in an amount between 1 and 30 % of the total weight of the composition.
f) A emollient base in an amount between 30 and 80% of the total weight of the composition, containing one or a mixture of the compounds selected from the group consisting in Vaseline, liquid parafine, wax, lanoline, olive oil, almond oil, karite oil, peanut oil, retinoic acid, isopropyl miristate, isopropyl palmitate, castor oil, calendula oil, glycerine, propyleneglycol, butyleneglycol, polyetileneglycol, coconut oil, medium chain tryglicerides and fish oil
g) Water in an amount lower than 10% of the total weight of the composition.

23. The composition according the claim 22 **characterised** wherein the rubefacient, antiprurigenous is camphora or a Cinnamomum camphora obtained by natural, semi synthesis or chemical synthesis, means or a mixture of them.

24. The composition of the claim 22 wherein the antiseptic is benzalconium chloride in an amount comprised between 0.02 and 1 %.

25. The composition of the claim 22 wherein the thickening agent comprises one or more of the compounds of the group formed by Aluminium stearate, corn starch, potato starch, rice starch, wheat starch, carboximethilcelulose, ethilcelulose and polyethyleneglycol.

26. The composition of the claim 22 wherein the water content is lower than 1 % of the toal weight.

27. A composition according any of the claims 22 to 26 that is applied on the wounds of animals belonging to the group of bovine, equine, swine, ovine, birds, cows, calf, pigs, horses, mules, donkeys, zebras, sheeps, goats, rodents, cats, dogs, ornamental birds, exotic birds, reptiles, poultry, ducks, rabbits, canary, parrots, parakeets, cockatoo.

28. A composition according any of the claims 22 to26 wherein is applied on the udders of cows, sheeps and goats.

29. A composition according any of the claims 22 to 28 wherein the withdrawal period in livestock animals is 0 days.

30. A method of prophylaxis and treatment of wounds of the skin of animals that comprises the step of the application of an effective amount of the composition according any of the claims 22 to 26 on the area to be treated.

31. The method of the claim 30 wherein the area to be treated is the udder or the hoof of the animal

32. The method of the claim 30 wherein the composition is extended on the affected area forming a protecting film.

33. The use of a composition according any of the claims 22 to 26 for the preparation of products to be used in the protection, cicatrisation, disinfection or treatment of wounds of the skin of the animals.

34. The use of a composition according any of the claims 22 to 26 wherein is added a colouring agent, perfume, a preservative or a mixture of them.

35. A composition according any of the claims 22 to 26 wherein is added an antioxidant.

36. The composition according the claim 35 wherein the antioxidant is buthylhidroxitoluene (BHT), butfylhidroxianisol (BHA), tocopherol or a mixture of them.

37. A method of manufacture of the composition according any of the claims 22 to 26 that comprises the mixture by means of stirring of the mentioned ingredients and the use of heat.

38. The method of the claim 37 wherein the filling is carried out by use of heat.

39. The method of the claim 37 wherein the product is prepared aseptically.

40. The method of the claim 37 wherein the product undergoes a process of final sterilisation or reduction of bioburden by means of use of heat, irradiation or gases.

41. A composition according any of the claims 22 to 26 that comprises the addition of balsam of Peru, Centella asiatica or an extract, aloe or an extract, or a mixture of all them.

42. A composition according any of the claims 22 to 26 **characterised** to be used for the treatment or prophylaxis of inespecific dermatitis or caused by ecto parasites in dogs and cats.
